# EUROPEAN PATENT APPLICATION

(11) **EP 1 870 091 A1**
(43) Date of publication of application: **26.12.2007**
(21) Application number: 06730431.1
(22) Date of filing: 29.03.2006
(51) Int. Cl.: A61K 9/127, A61K 39/00, A61K 47/26, A61K 47/36, A61P 35/00, A61P 37/04

(54) **LIPOSOME COMPOSITION FOR INDUCTION OF IMMUNITY**

(30) Priority: 29.03.2005 JP 2005094380
(71) Applicant: TOKAI UNIVERSITY EDUCATIONAL SYSTEM, Shibuya-ku Tokyo 1510063 (JP); Aichi Prefecture, Nagoya-shi, Aichi 460-0001 (JP)
(72) Inventor: KOJIMA, Naoya, 0813 (JP); IKEHARA, Yuzuru, anokoden, Chikusa-ku, Nagoya-shi, Aichi, (JP); TSUJIMURA, Kunio, anokoden, Chikusa-ku, Nagoya-shi, Aichi, (JP)
(74) Representative: Polypatent
(86) International application number: PCT/JP2006/306483
(87) International publication number: WO 2006/104199

(57) **Abstract**

It is an object of the present invention to provide a liposome composition which is able to allow the MHC class I and class II molecules of antigen-presenting cells to efficiently present an antigen substance. The present invention provides a liposome composition, which comprises an oligosaccharide-coated liposome and an antigen substance and is used to cause the MHC class I and class II molecules of an antigen-presenting cell to present an antigen peptide.

## Description

### Technical field

The present invention relates to a liposome composition for immune induction, in which an oligosaccharide-coated liposome is used. More specifically, the present invention relates to a liposome composition using an oligosaccharide-coated liposome, which is characterized in that it is incorporated into a macrophage existing in an abdominal cavity when it is administered into the abdominal cavity and that it is presented on MHC class I and class II molecules.

### Background Art

The number of deaths from stomach cancer in the total number of deaths from all types of cancers is second highest, following that from lung cancer. The major factor is disseminated metastasis to various organs in an abdominal cavity. Accordingly, in order to recover from stomach cancer, it is essential to develop an immunotherapy that is able to control metastasis to peritoneum and the progression thereof.

Major effector cells existing in an immune system that reject cancers are cytotoxic T lymphocytes (CTL). In order to exhibit the functions of such cells, helper T cells (Th) play an important role. Thus, in order to induce an efficient immune reaction, it is considered essential that the two cell groups be simultaneously activated. An immunotherapy for cancers, which uses both a helper (Th) epitope and a cytotoxic T lymphocyte (CTL) epitope as antigens, has been examined.

As stated above, in order to achieve an efficient immune reaction, it is necessary to simultaneously activate helper T cells (Th) and cytotoxic T lymphocytes (CTL). For such simultaneous activation, it is ideal to conduct immunization using an antigen comprising both a Th epitope (MHC class II) and a CTL epitope (MHC class I). However, it has been known that it is difficult to efficiently activate CTL by immunization with an intact protein. It has been generally known that an endogenous antigen is presented on MHC class I, whereas an exogenous antigen is presented on MHC class II. However, it is said that an exogenous antigen incorporated into an antigen-presenting cell such as a macrophage is presented on MHC class II more easily than on MHC class I. That is, when an antigen is used as a vaccine and is singly inoculated into a subject for immunization, CTL, which depends on antigen presentation by MHC class I molecules, is not efficiently activated.

Since major effector cells existing in an immune system that reject cancers are CTL, several attempts have been made to allow class I molecules to present an exogenous antigen. Such attempts, which have been made as immunotherapy, include: (1) a method which comprises collecting antigen-presenting cells from a patient, culturing them, adding an antigen peptide to the culture, and returning the resultant culture to the patient; and (2) a method which comprises introducing an antigen gene to antigen-presenting cells. The two above methods have been largely problematic in terms of technical, ethical, and economical aspects. Moreover, it is necessary to identify a cancer antigen that is suitable for immunization. Further, in order to simultaneously activate several numbers of Th/CTL by the aforementioned method (1), it is also necessary to identify both the Th and CTL epitopes.

Furthermore, with regard to a polysaccharide-conjugated liposome, it has been revealed that pullulan and mannan are incorporated into a macrophage, and that they are presented on MHC class I molecules. In immunotherapy, a method of conjugating an antigen to such polysaccharides and administering it to a subject has been attempted. However, since such a polysaccharide structure includes antigenecity or toxicity, it cannot be necessarily said that application of such a method to a human is safe. Further, it has not yet been proved that MHC class II molecules are able to present such an antigen.

### Disclosure of the Invention

### An object to be Solved by the Invention

It is an object of the present invention to solve the aforementioned problems of the prior art techniques. In other words, it is an object of the present invention to provide a liposome composition which is able to allow the MHC class I and class II molecules of antigen-presenting cells to efficiently present an antigen substance.

### A Means for Solving the Object

As a result of intensive studies directed towards achieving the aforementioned object, the present inventors have found that an antigen can be delivered to a macrophage by encapsulating such an antigen into an oligomannose-coated liposome (MCL) and administering it to a subject. This is because of a reaction that is mediated by a mannose receptor expressed by the macrophage. Such a macrophage specifically and actively incorporates a liposome that has been administered into an abdominal cavity, and becomes activated. In the subsequent immune reaction, the macrophage presents the encapsulated antigen on its own MHC class I and II molecules, and it migrates to the extranodal lymphatic tissues of greater omentum or mesentery, so as to activate cellular immunity. At that time, the macrophage is able to present a peptide derived from the encapsulated antigen on both the MHC class I and class II molecules, and it also migrates to a regional lymph node. In an experiment using mice, a macrophage, which incorporates MLC that has been administered into an abdominal cavity, becomes activated, and reaches greater omentum acting as a regional lymph node. At the same time, the macrophage presents the peptide derived from the encapsulated antigen on both the MHC class I and class II molecules, and it activates the two cell groups, Th and CTL, so as to allow them to generate IFN-γ. The present invention has been completed based on such findings.

Thus, the present invention provides a liposome composition, which comprises an oligosaccharide-coated liposome and an antigen substance and is used to cause the MHC class I and class II molecules of an antigen-presenting cell to present an antigen peptide.

The oligosaccharide is preferably oligomannose.

The oligosaccharide is preferably mannopentaose or mannotriose.

The antigen substance is preferably a cancer antigen.

Preferably, the liposome composition of the present invention is administered into an abdominal cavity, subcutis, or intranasal mucosa, and it is incorporated into an antigen-presenting cell such as a macrophage, and as a result, an antigen peptide is presented on the MHC class I and class II molecules.

The liposome composition of the present invention is preferably used to induce cytotoxic T lymphocytes (CTL).

### Best Mode for Carrying Out the Invention

The embodiments of the present invention will be specifically described below.

The present inventors have revealed that when an ovalbumin (OVA) is used as a model antigen, and when such an exogenous antigen protein is encapsulated into an oligomannose-coated liposome (MCL) and is then administered to a subject, the antigen can be presented not only on the MHC class II, but also on the MHC class I. When an ovalbumin (OVA) or an OVA peptide was encapsulated into a mannose-coated liposome and such a liposome was then directly administered into an abdominal cavity, it was quickly incorporated into a macrophage. Thereafter, the macrophage was recovered from the abdominal cavity and was then cultured for 24 hours. Subsequently, the obtained culture was subjected to a mixed culture together with splenic CD8-positive T-lymphocytes derived from a transgenic (Tg) mouse OT-1, into which a T cell receptor gene specific to the OVA peptide presented on the class I molecules had been introduced. As a result, when OVA dissolved in PBS was administered into the abdominal cavity of a mouse and macrophages were then recovered from the mouse, almost no IFN-γ was generated. However, when OVA was encapsulated into an oligomannose-coated liposome, IFN-γ was generated. This result clearly showed that after OVA-encapsulated MCL had been incorporated into macrophages, the OVA peptide was presented on the MHC I class molecules. On the other hand, even in the case of directly administering OVA, such OVA was also incorporated into macrophages. However, IFN-γ was not generated. Thus, it was considered that the OVA peptide was not presented on the MHC class I molecules. Accordingly, it has been demonstrated that an antigen can be efficiently presented on the MHC class I molecules of antigen-presenting cells by encapsulating an antigen protein or an antigen peptide into MCL and then administering the MCL.

On the other hand, when the above culture was subjected to a mixed culture together with splenic C4-positive T-lymphocytes derived from a transgenic (Tg) mouse OT-2, into which a T cell receptor gene specific to the OVA peptide presented on the class II molecules had been introduced, generation of IFN-γ that was specific to the OVA peptide presented on the MHC class II molecules was induced. Thus, it was demonstrated that an antigen was efficiently presented on the aforementioned molecules in both cases where the antigen was encapsulated into an oligomannose-coated liposome and where the antigen was directly inoculated.

In addition, when a soluble protein of mouse EL4 lymphoma that had been encapsulated into MCL was subcutaneously administered to a syngeneic mouse, strong CTL activity to EL4, which had hardly been observed when a solubilized product had been directly administered, was observed. That is, this result showed that even when an antigen is subcutaneously administered, it is incorporated into an antigen-presenting cell, and that the antigen peptide is efficiently presented on the MHC class I molecules. This result also showed that even if an antigen protein has been unknown or has not yet been purified, a peptide capable of inducing CTL can be presented on the MHC class I molecules by encapsulating an extract from cancer cells into a mannose-coated liposome and then administering the above liposome. Thus, it was demonstrated that the use of such a liposome is effective as a means for obtaining vaccine effects upon cancer.

The liposome composition of the present invention can also be used as a drug delivery system. The term "drug delivery system" is used herein to mean a system for delivering drugs. In general, when a drug is administered, it is spread throughout the body. The drug delivery system is a technique of preventing such dispersion of a drug and delivering it to the targeted tissues or cells. This drug delivery system has the effect of reducing side effects or improving drug effect.

The term "liposome" is used in the present invention to mean an artificial vehicle that is made of a double membrane constituted with phospholipids. A hydrophilic part is oriented on the inside of the membrane, and a hydrophobic part is oriented on the outside thereof. A hydrophilic compound can be encapsulated into the lumen thereof. The liposome composition of the present invention can be used in a drug delivery system for anticancer agents and the like.

The liposome composition of the present invention is characterized in that it comprises an oligosaccharide-coated liposome and an antigen substance. The above liposome composition is used to cause the MHC class I and class II molecules of an antigen-presenting cell to present an antigen peptide derived from the antigen substance. More specifically, when the liposome composition of the present invention is administered into an abdominal cavity, it is incorporated into an antigen-presenting cell such as a macrophage, and an antigen peptide is presented on the MHC class I and class II molecules of the aforementioned cell.

The macrophage is a cell having phagocytosis. This cell recognizes foreign substances that do not exist in a body, nearly dead (apoptotic) cells that do not constitute a living body any more, cancer cells, etc., and it incorporates such cells therein and then digests them. Thereafter, the macrophage particularly presents a peptide fragment of the digested protein, which will be a target of attack from an immune system, on the cell surface thereof, and functions as a control tower for inform an immune network of the target of attack.

As an oligosaccharide-coated liposome used in the present invention, the liposome described in Japanese Patent No. 2828391 can be used. The type of a sugar component constituting such an oligosaccharide is not particularly limited. Examples of such a sugar component include D-mannose (D-Man), L-fucose (L-Fuc), D-acetylglucosamine (D-GlcNAc), D-glucose (D-Glc), D-galactose (D-Gal), D-acetylgalactosamine (D-GalNAc), and D-rhamnose (D-Rha).

In an oligosaccharide, individual constituent sugars bind to one another via an α1→2 bond, an α1→3 bond, an α1→4 bond, an α1→6 bond, a β1→4 bond, or a combination thereof. For example, mannose may constitute a single strand via the aforementioned bond, or may adopt a branched structure via the combination of an α1→3 bond with an α1→6 bond. The number of monosaccharides contained in an oligosaccharide is preferably 2 to 11. Specific examples of such an oligosaccharide include mannobiose (Man2), mannotriose (Man3), mannotetraose (Man4), mannopentaose (Man5), mannohexaose (Man6), mannoheptaose (Man7), and various types of mixed oligosaccharides such as M5 (Chemical formula 1) and RN (chemical formula 2) as shown below. 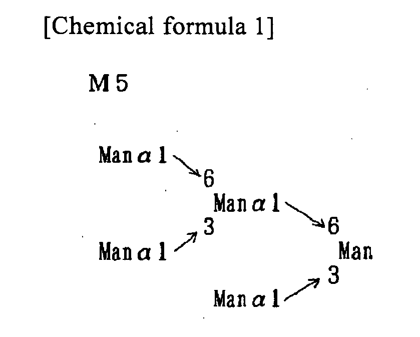 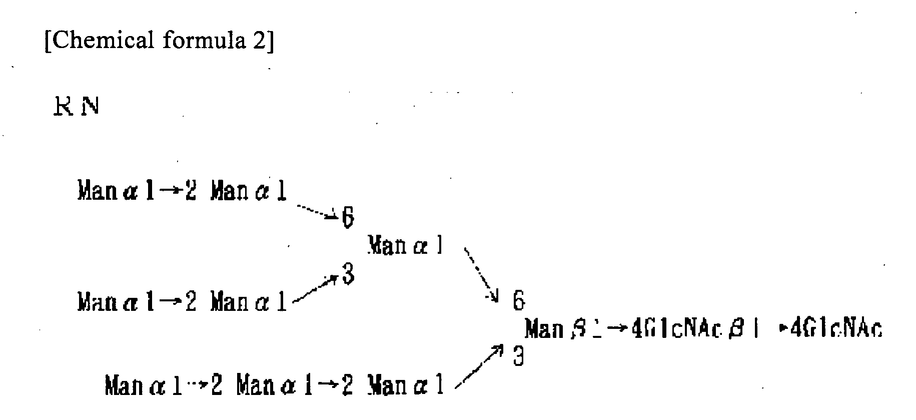 (wherein each Man that binds to one another via an α1→2 bond independently may be present or may not be present.)

An oligosaccharide having the structure represented by chemical formula 3 is an example of an oligosaccharide that contains glucose. An oligosaccharide having the structure represented by chemical formula 4 is an example of an oligosaccharide that contains N-acetylglucosamine. An oligosaccharide having the structure represented by chemical formula 5 is an example of an oligosaccharide that contains fucose. 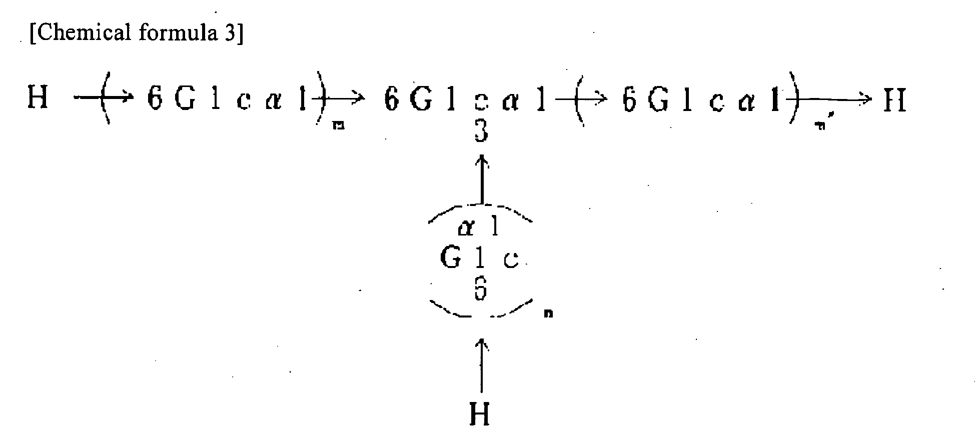 (wherein m + m' + n is 1 to 10.)

[Chemical formula 4] GlcNAcβ1 (>4GlcNAcβ1), > 4GlcNAc

(wherein n is 0 to 4.) 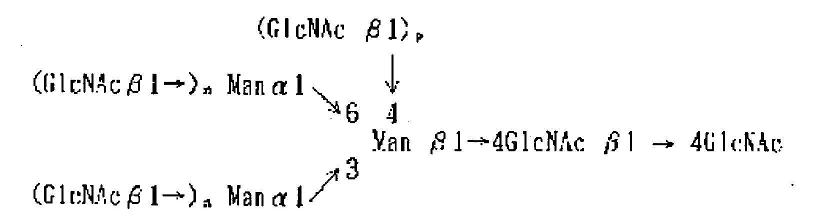 (wherein p is 0 or 1; each n is independently 0 to 3; two GluNAc residues represented by 4GlcNAcβ1→4GlcNAc on the right hand side in the formula may be or may not be present independently from each other; and all of GlcNAc represented by (GlcNAcβ1→)ₙ may bind to any portion of a free hydroxyl group of Man located on the right side thereof via a glycoside bond.) 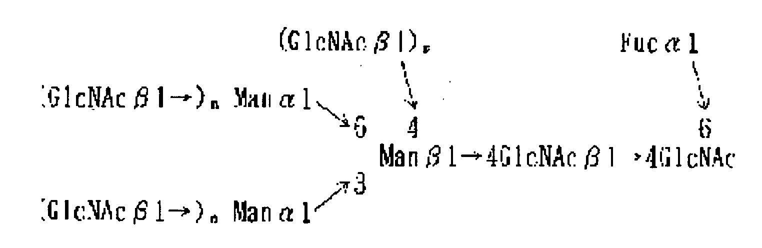 (wherein p is 0 or 1; each n is independently 0 to 3; and all of GlcNAc represented by (GlcNAcβ1→)ₙ may bind to any portion of a free hydroxyl group of Man located on the right side thereof via a glycoside bond.) 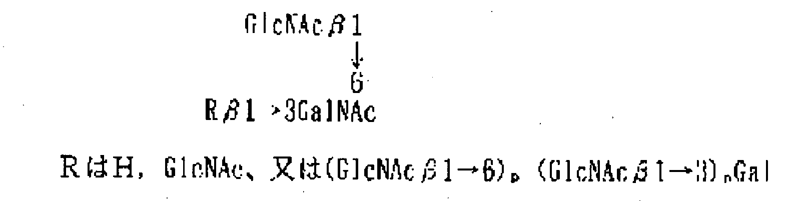 (wherein p is 0 or 1.) 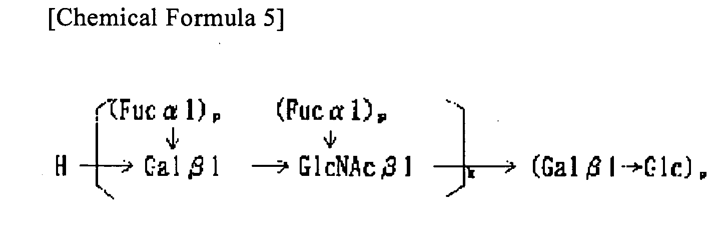 (wherein k is 1 to 5; each p is independently 0 or 1; and a component having no destination number at the tip of the arrow may bind to any portion of a free hydroxyl group via a glycoside bond.) 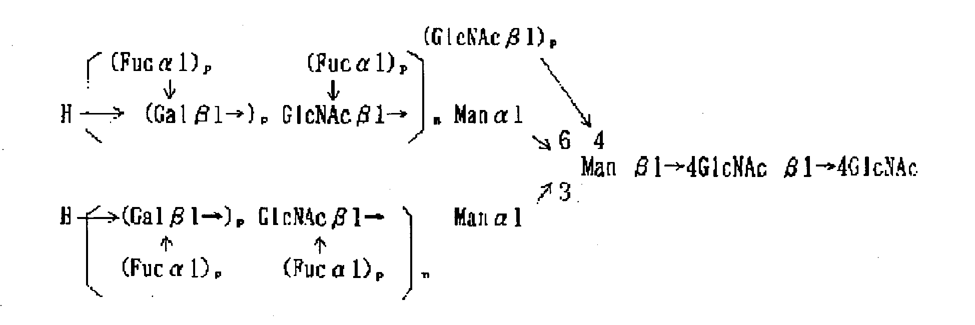 (wherein each p is independently 0 or 1; each n is independently 0 to 3; a component having no destination number at the tip of the arrow may bind to any portion of a free hydroxyl group via a glycoside bond; and two GluNAc residues represented by 4GlcNAcβ1→4GlcNAc on the right side in the formula may be or may not be present independently from each other.) 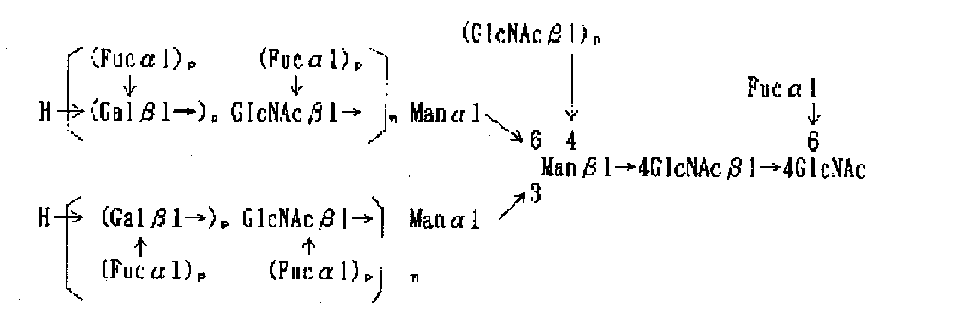 (wherein each p is independently 0 or 1; each n is independently 0 to 3; a component having no destination number at the tip of the arrow may bind to any portion of a free hydroxyl group via a glycoside bond; and two GluNAc residues represented by 4GlcNAcβ1→4GlcNAc on the right side in the formula may be or may not be present independently from each other.)

The oligosaccharide used in the present invention is preferably oligomannose, and particularly preferably mannopentaose or mannotriose.

All of the aforementioned oligosaccharides have a reducing end aldehyde group. Thus, such an aldehyde group can be used as a means for introducing an oligosaccharide into the surface of a liposome. Namely, such aldehyde is allowed to react with a lipid having an amino group to form a Schiff base. Subsequently, according to a common method, such a Schiff base is reduced, and is preferably chemically reduced by NaBH₃CN, for example, so as to bind the oligosaccharide to the lipid (Tsuguo Mizuochi, Toshitsu-kodaku (Carbohydrate Engineering), pp. 224-232, Biotechnology Information Center, Industrial Research Center of Japan, 1992).

The aforementioned lipid having an amino group is preferably a phospholipid having an amino group. For example, phosphatidylamine such as dipalmitoylphosphatidylethanolamine (DPPE) or distearoylphosphatidylethanolamine (DSPE) can be used. The thus obtained bound substance of an oligosaccharide and a lipid may also be referred to as an artificial glycolipid in the present invention.

As a lipid that constitutes a liposome, commonly used any given lipids, which have been known as components of a liposome, may be used singly or as a mixture of several lipids. For example, natural lipids such as egg yolk, soybean, or lipids obtained from other types of animals and plants, modified lipids obtained by modification of the aforementioned lipids, such as lipids whose unsaturation degree is decreased by hydrogenation, or chemically synthesized lipids, can be used. Specific examples of such lipids include: sterols such as cholesterol (Chol); phosphatidylethanolamines such as dipalmitoylphosphatidylethanolamine (DPPE) or distearoylphosphatidylethanolamine (DSPE); phosphatidylcholines such as dipalmitoylphosphatidylcholine (DPPC) or distearoylphosphatidylcholine (DSPC); phosphatidylserines such as dipalmitoylphosphatidylserine (DPPS) or distearoylphosphatidylserine (DSPS); and phosphatidic acids such as dipalmitoylphosphatidic acid (DPPA) or distearoylphosphatidic acid (DSPA).

A liposome can be produced according to a known method [D. W. Deeamer, P. S. Uster, "Liposome" ed. by M. J. Ostro, Marcel Dekker Inc., N. Y. Basel, 1983, p. 27]. A vortex method and an ultrasonic wave method are common. Other than the aforementioned methods, an ethanol injection method, an ether method, and a reverse phase evaporation method can also be applied. Such methods can also be used in combination.

For example, in the case of the vortex method and the ultrasonic wave method, a certain lipid is dissolved in an organic solvent such as methanol, ethanol, chloroform, or a mixture thereof including a mixture of methanol and chloroform. Thereafter, the aforementioned organic solvent is eliminated by evaporation, so as to obtain a thin layer of lipid. Subsequently, an aqueous vehicle is added to the thin layer of lipid, followed by a vortex treatment or ultrasonication, so as to form a liposome. During this operation, a substance to be administered, such as a drug, a marker, or a contrast medium, is mixed into the aforementioned aqueous vehicle. For example, such a substance is dissolved or suspended in the aforementioned aqueous vehicle, so that the substance to be administered can be encapsulated into a liposome.

In order to introduce an oligosaccharide into the surface of a liposome, either one of the following two methods may be used, for example. When the aforementioned artificial glycolipid is water-soluble and is not sufficiently dissolved in an organic solvent, for example when a bound substance of the aforementioned M5 and DPPE (M5-DPPE) or a bound substance of RN and DPPE (RN-DPPE) is used, an aqueous solution that contains such a bound substance is prepared, and the aqueous solution is then mixed with the formed liposome. Thereafter, the obtained mixture may be incubated at a temperature from 4°C to room temperature for 24 to 120 hours, for example, for approximately 72 hours.

On the other hand, when the aforementioned artificial glycolipid is dissolved in an organic solvent, the artificial glycolipid, together with a lipid that is used to constitute a liposome, is dissolved in the aforementioned organic solvent in the production process of a liposome. Thereafter, a liposome may be formed according to a common method. The amount of an oligosaccharide used to produce a liposome differs depending on the type of the oligosaccharide, the type of an antigen to be encapsulated, the combinational structure of a liposome, etc. In general, 5 to 500 µg of oligosaccharide is used with respect to 1 mg of lipid that constitutes a liposome.

The liposome used in the present invention may be either of a multilamellar type (multilamella vehicle) or of a unilamellar type (unilamella vehicle). Such a liposome can be prepared by a common known method. In addition, it is also possible that one type can be converted to the other type according to a common method. For example, a liposome of a multilamellar type can be converted to a liposome of a unilamellar type. The particle size of the liposome used in the present invention is not particularly limited. If necessary, the particle size of the liposome can be adjusted according to a common method, for example, by filtration with a filter having a desired pore size.

In the present invention, an oligomannose-coated liposome is particularly preferably used. Such an oligomannose-coated liposome is obtained by lipidating several mannose sugar chains (oligomannose) that constitute a sugar chain structure that is widely conserved in organisms ranging from yeast to a human, purifying it, and then conjugating the resultant to a liposome. Such an oligomannose-coated liposome is not toxic because a structure that is originally present in a human body is used. When a receptor that exists in a macrophage specifically recognizes oligomannose, an oligomannose-coated liposome is immediately incorporated into the cell due to phagocytosis.

The type of the antigen substance used in the present invention is not particularly limited. Examples of such an antigen substance include survivin, livin, rikavarin, gp110, MART-1, NY-ESO-1, SSX, PBF, HER2, SYT-SSX, CEA, and MUC-1. Such an antigen substance is preferably a cancer antigen.

The amount of an antigen substance contained in a liposome is not particularly limited, as long as the effect of the present invention, such that the administered liposome composition is incorporated into a macrophage existing in an abdominal cavity and then that an antigen peptide is presented on the MHC class I and class II molecules of the antigen-presenting cell, can be obtained. The amount of such an antigen substance may be appropriately determined, depending on the type of a substance to be administered, the composition of a liposome, a structure thereof, etc. In general, 1 to 100 µg of antigen substance is administered with respect to 1 mg of lipid that constitutes a liposome.

The liposome composition of the present invention may comprise a pharmaceutically acceptable carrier, as desired. Examples of a carrier used herein include sterilized water, buffer solution, and saline. In addition, the liposome composition of the present invention may also comprise salts, sugars, proteins, starch, gelatin, vegetable oil, polyethylene glycol, etc.

The administration route of the liposome composition of the present invention is not particularly limited. The aforementioned liposome composition can preferably be administered into an abdominal cavity, subcutis, or intranasal mucosa. The dosage of the liposome composition of the present invention differs depending on the type of a substance to be administered, an administration route, the severity of symptoms, the age and condition of a patient, the degree of side effects, etc. In general, the aforementioned liposome composition is administered at a dosage of 0.1 to 100 mg/kg/day.

The present invention will be more specifically described in the following examples. However, these examples are not intended to limit the scope of the present invention.

### Examples

### Example 1: Method of producing oligosaccharide-coated liposome and method of encapsulating antigen therein

According to the following method, mannotriose (M3) (mannotriose (Man3) having a structure of Manα1→6 (Manα1→3) Man) was allowed to chemically bind to dipalmitoylphosphatidylethanolamine (DPPE) via a reductive amination reaction, so as to synthesize M3-DPPE.

First, 600 µl of distilled water was added to 2.5 mg of mannotriose (M3), and the reaction solution was stirred, so that the above substance was dissolved in the distilled water, thereby preparing an oligosaccharide solution. Subsequently, DPPE was dissolved in a concentration of 5 mg/ml to a mixed solution of chloroform/ethanol (1 : 1; volume ratio), so as to prepare a DPPE solution. In addition, NaBH₃CN was dissolved in a concentration of 10 mg/ml in methanol, so as to prepare a NaBH₃CN solution. 9.4 ml of the aforementioned DPPE solution and 1 ml of the aforementioned NaBH₃CN solution were added to 600 µl of each of the aforementioned oligosaccharide solutions, and the three types of solutions were blended by stirring. The reaction mixture was incubated at 60°C for 16 hours, so as to generate an artificial glycolipid. The synthesized artificial glycolipid was purified by HPLC, so as to obtain a highly purified glycolipid.

A liposome, into which an antigen protein (ovalbumin (OVA) or EL4 antigen) had been encapsulated, was produced as follows.

First, a chloroform/methanol solution or an ethanol solution, which contained dipalmitoylphosphatidylcholine (DPPC), cholesterol, and an artificial glycolipid (M3-DPPE) that had been mixed at a ratio of 1 : 1 : 0.1, was placed in a pear-shaped flask. The mixture was dried using a rotary evaporator under reduced pressure, so as to produce a lipid film. Subsequently, 0.3 ml of a PBS solution (5 mg/ml) that contained an antigen protein was added to the lipid film, and the obtained mixture was then stirred using a vortex mixer, so as to produce an M3-DPPE-coated liposome (OML).

Thereafter, the liposome was washed with PBS several times, and soluble substances that had not been encapsulated into the liposome were eliminated by centrifugation. Moreover, the particle size of the liposome was adjusted using a 1-µm filter. The amount of a protein encapsulated was quantified by protein quantification. In addition, the composition ratio of the lipid in the liposome and the amount of a drug were quantified by HPLC.

### Example 2: Confirmation of antigen presentation to MHC class I and class II molecules

Ovalbumin (OVA) or an OVA peptide was encapsulated into a mannose-coated liposome, and the liposome was then directly administered into the abdominal cavity of a C57BL/6 mouse or a BALB/c mouse. The liposome was rapidly incorporated into a CD 11b-positive cell, which expressed both the MHC class I, and the MHC class I and class II. It has been known that such a CD11b-positive cell functions as an antigen-presenting cell. Thus, for the purpose of demonstrating that OVA can be presented on both the MHC class I and class II molecules after an OVA-encapsulated oligomannose (M3)-coated liposome has been incorporated into such a CD11b-positive cell, the following experiment was carried out.

### (1) Experimental groups

In group A, 50 µg of OVA dissolved in PBS had been encapsulated into an M3-coated liposome (OML). PBS was added to the liposome, resulting in a total volume of 200 µl. The obtained solution was administered into the abdominal cavity of each mouse.

In group B, OVA was dissolved in PBS in a concentration of 5 mg/ml. To 10 µl of such an OVA solution, PBS was added, resulting in a total volume of 200 µl. The obtained solution was administered into the abdominal cavity of each mouse. In group B also, 50 µg of OVA was administered to the mouse, as in the case of group A.

In group C, PBS was added to 38 µl of an M3-coated liposome, into which only PBS had been encapsulated, resulting in a total volume of 200 µl. The obtained solution was administered into the abdominal cavity of each mouse.

In group D, 200 µl of PBS was administered into the abdominal cavity of each mouse.

### (2) Pre-treatment of CD 11b-positive cell recovered from abdominal cavity

Eight-week-old C57BL/6 or BALB/c mice were used. The abdominal part of each mouse was disinfected with an absorbent cotton immersed in 70% ethanol. Thereafter, each of the aforementioned solutions was then administered to each group using a syringe for tuberculinization. One hour after the administration, mice were subjected to euthanasia by administration of Nembutal as an anesthetic agent. Thereafter, 5 ml of Hank's balanced salt solution was quickly injected into the abdominal cavity of the mouse to wash it, and the aforementioned solution was then recovered therefrom, so as to recover free cells existing in the abdominal cavity. The cells, which had been recovered from 3 mice of each group using the Hank's balanced salt solution, were gathered for every group, and the thus gathered cells were then centrifuged at 1,000 rpm for 5 minutes. The precipitated cells were suspended in 10 ml of RPMI solution (RPMI-A solution) that contained 10% fetal bovine serum, and they were then centrifuged at 1,000 rpm for 5 minutes again. The same above operation was repeated twice, and the resultant cells were suspended in RPMI solution (RPMI-B solution) that contained 1 µM 2-mercaptoethanol and 10% fetal bovine serum. 100 µl of the suspension was dispensed into a 96-well plate. The cells were cultured in a CO₂ incubator at 37°C at 5% CO₂ for 2 hours, so that CD11b-positive cells could be adhered. Non-adhered cells were washed out, and the remaining cells were cultured in 100 µl of RPMI-B solution for 24 hours.

### (3) Detection of antigen presented on MHC class I and class II molecules

The presence or absence of an OVA peptide presented on the MHC class I or class II molecules of C57BL/6 mice was detected using mice of C57BL/6 line, namely, OT-1 mice (which recognize OVA₂₅₇₋₂₆₄ presented on H-2K^{b} as an MHC class I molecule) and OT-2 mice (which recognize OVA₃₂₃₋₃₃₉ presented on H-2A^{b} as an MHC class II molecule), respectively. A spleen was excised from each of such mice, and it was ground with two pieces of slide grasses, while the spleen was immersed in 5 ml of Hank's balanced salt solution. The thus ground spleen was transferred into a 15-ml centrifuge tube and was then left at rest for 5 minutes to precipitate fibrous tissues that constituted the splenic tissues. The precipitated fibrous tissues were eliminated. Thereafter, the residue was centrifuged using the mouse lymphocyte separation medium, M-SMF (JIMRO Co., Ltd.) to obtain lymphocytes. In addition, an experiment was carried out to examine the efficiency of antigen presentation by the class II molecules, using DO11.10 mice (which recognize an OVA peptide presented by H-2A^{d}/OVA₃₂₃₋₃₃₉) that were mice of BALB/c line. 1 x 10⁷ lymphocytes were suspended in 1 ml of RPMI-B solution, and 100 µl each of the suspension was added to each well that contained the prepared CD11b-positive cells (2-d), followed by culture. 48 hours later, the culture supernatant was recovered, and the value of IFN-γ contained in the supernatant was quantified.

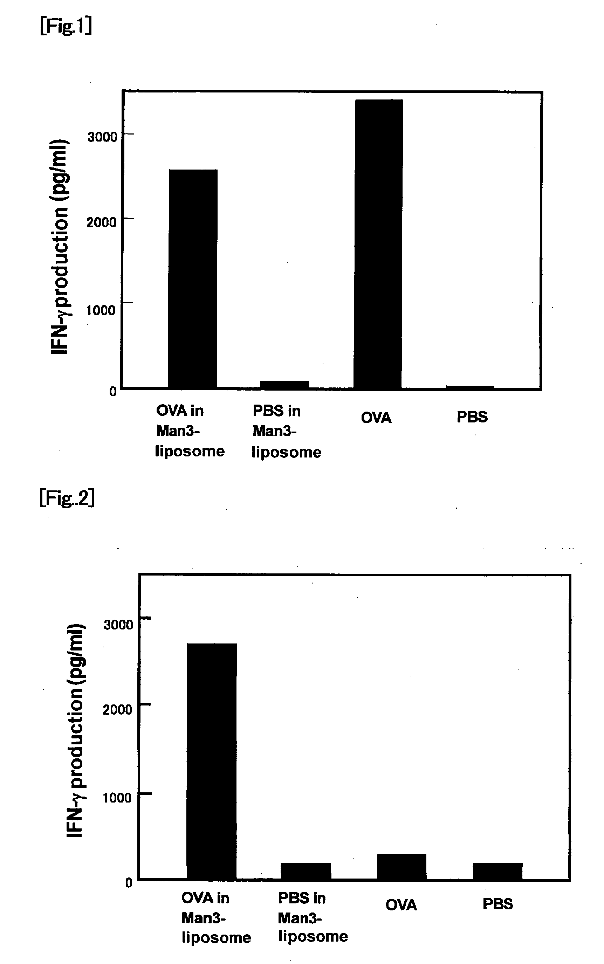

The results are shown in Figures 1 and 2. The results as shown in Figures 1 and 2 demonstrated that when ovalbumin (OVA) encapsulated into a mannose-coated liposome is administered to a subject, an antigen is presented on both the MHC class I and class II molecules, and IFN-γ is efficiently induced.

### Example 3: Induction of CTL to mouse lymphoma EL4

EL4 cells were administered into the subcutis of a C57BL/6 mouse that was a syngeneic mouse, so as to obtain an EL4 cell mass. This cell mass was homogenized in PBS, and 100,000 g of the obtained supernatant was used as an EL4 antigen.

An EL4 antigen was encapsulated into an oligomannose-coated liposome, and 1 µg of protein was administered into the subcutis of a C57BL/6 mouse for immunization (total 3 times at intervals of 1 week).

Three weeks after the initial immunization, a spleen was excised from the mouse, and it was ground with two pieces of slide grasses, while the spleen was immersed in 5 ml of Hank's balanced salt solution. The thus ground spleen was transferred into a 15-ml centrifuge tube and was then left at rest for 5 minutes to precipitate fibrous tissues that constituted the splenic tissues. The precipitated fibrous tissues were eliminated. Thereafter, the residue was centrifuged using the mouse lymphocyte separation medium, M-SMF (JIMRO Co., Ltd.) to separate lymphocytes. The thus separated lymphocytes were used as effector cells. Thereafter, the effector cells were stimulated with a solubilized protein antigen (100 µg EL4/10⁷ cells) for 3 days. The effector cells were mixed with target cells (EL4 cells: 10⁴ cells/well) at the following ratios (E/T ratios = 50 : 1; 25 : 1; 12.5 : 1; 6.25 : 1; 3.125 : 1; and 1 : 1). Eight hours after the culture, cytotoxicity was measured with a CytoTox96 assay kit (Promega).

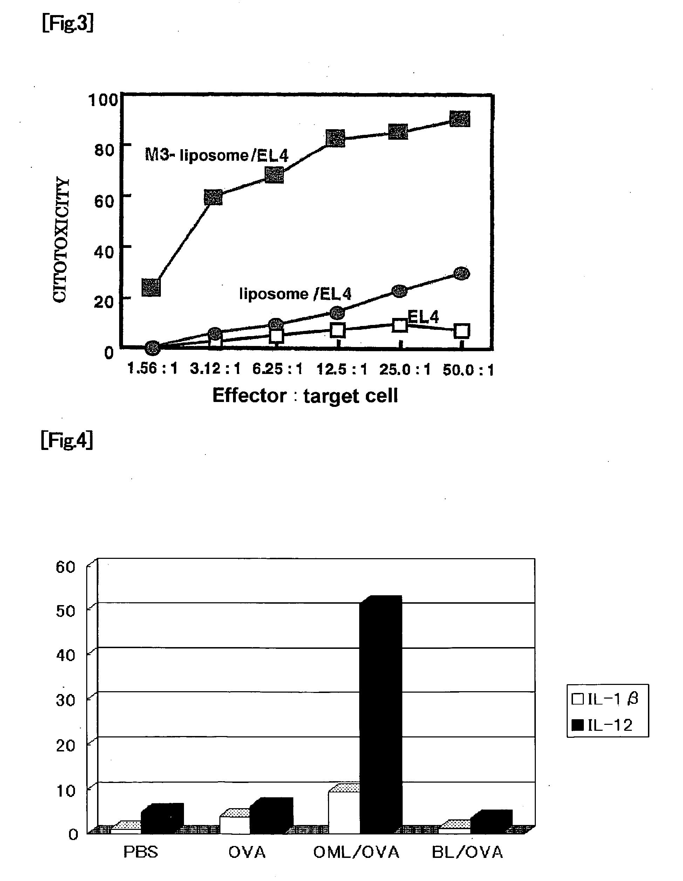

The measurement results are shown in Figure 3. As is clear from the results as shown in Figure 3, when an EL4 antigen encapsulated into an oligomannose-coated liposome was administered to a subject, CTL could be efficiently induced.

### Example 4:

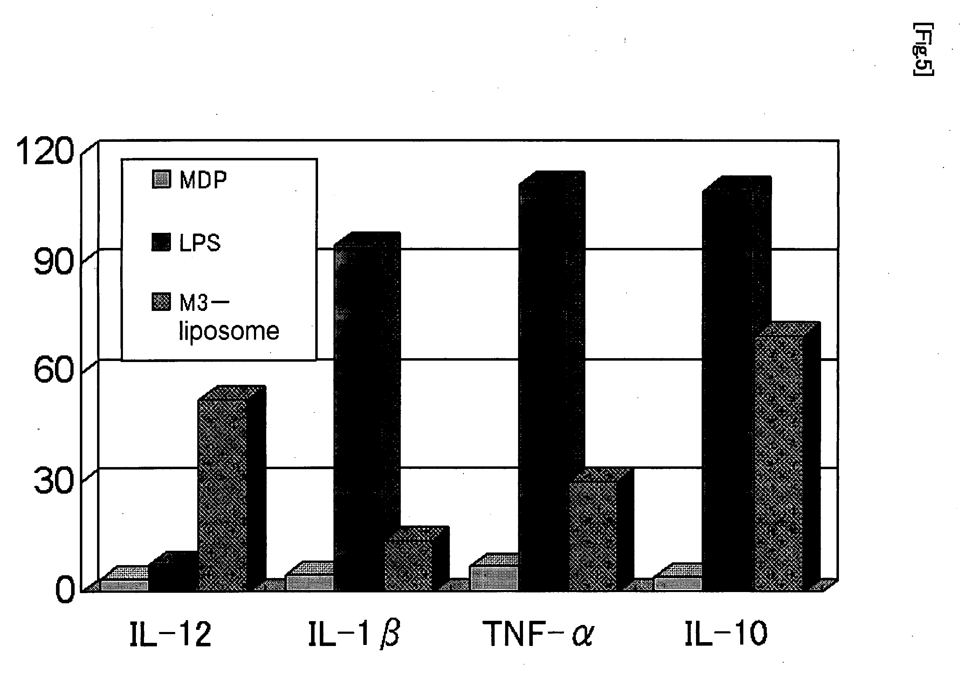

OVA-encapsulated OML (oligomannose-coated liposome, OML/OVA), OVA-encapsulated oligomannose-uncoated liposome (BL/OVA), or only OVA was administered into the abdominal cavity of a mouse (5 µg of OVA). 1 hour later, intraperitoneal cells were recovered and were then transferred to a petri dish used in culture, followed by culture for 1 hour. Thereafter, suspended cells were eliminated, and a fresh RPMI1640 medium was then added to adhered cells. 24 hours later, the medium was recovered, and cytokine contained in the medium was measured by the ELISA method. The results are shown in Figure 4. IL12 was generated only from OML/OVA.

### Example 5:

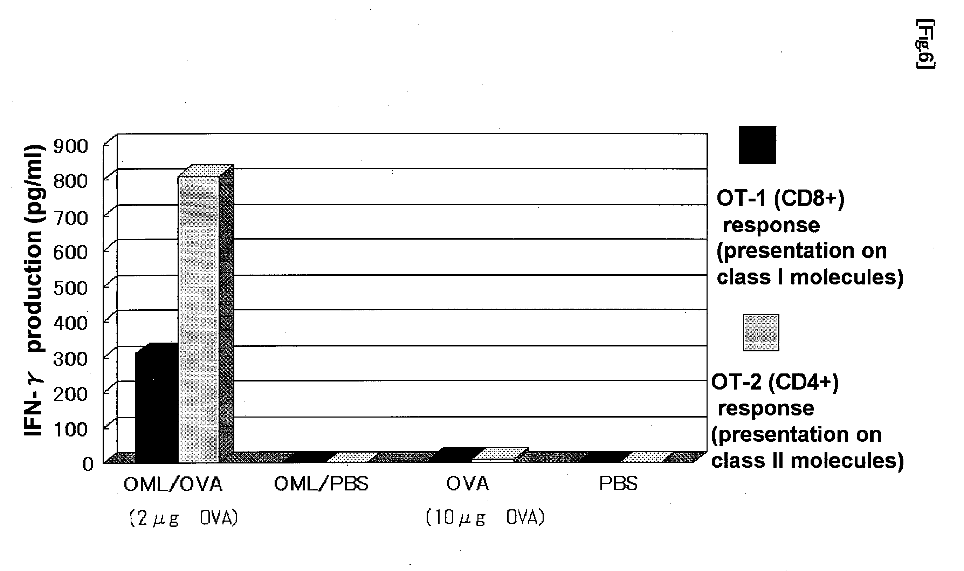

OML/OVA or LPS (10 ng) was administered into the abdominal cavity of a mouse in the same manner as in Example 4. 1 hour later, intraperitoneal cells were recovered, and the amount of cytokine generated from macrophages was then measured. The results are shown in Figure 5. IL1 and TNF were predominantly generated as a result of LPS stimulation, whereas IL12 was predominantly generated as a result of OML stimulation.

### Example 6:

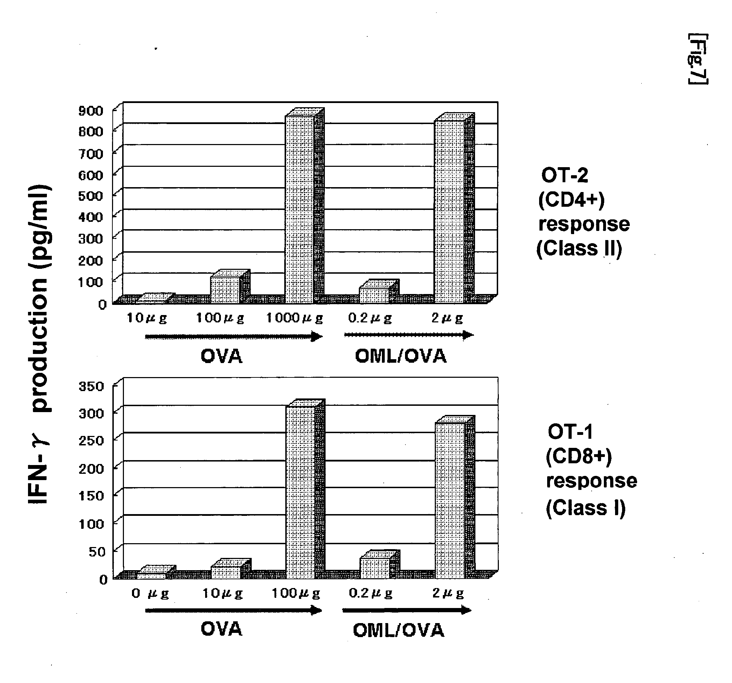

CD8-positive T cells and CD4-positive T cells were prepared from the transgenic mice OT-1 and the transgenic mice OT-2, which had been transfected with TCR that recognized OVA257-264 on H-2Kb (MHC class I) and TCR that recognized OVA323-339 on H-2Ab (MHC class II), respectively. The thus prepared cells were defined as responder cells. At the same time, OVA-encapsulated OML was administered into the abdominal cavity of a C57/BL6 mouse, and intraperitoneal cells were recovered 1 hour after the administration. The recovered cells were cultured, and adhered cells were then used as macrophages. The aforementioned T cells and macrophages were subjected to a mixed culture. 24 hours later, the culture solution was recovered, and the amount of cytokine contained in the medium was measured. The results are shown in Figure 6.

### Example 7:

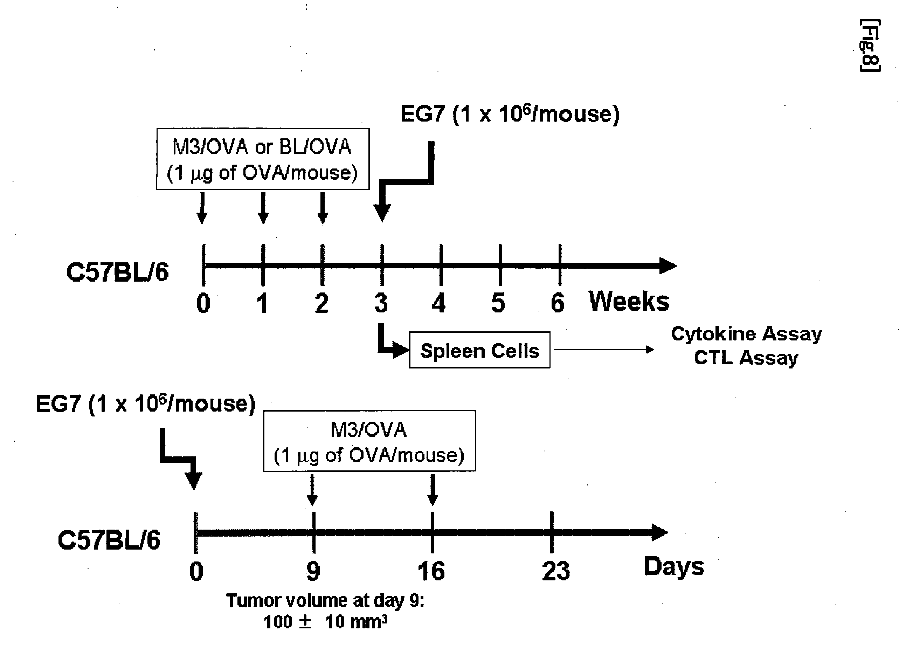

A single administration of OVA was compared with administration of OVA that had been encapsulated into OML, in terms of the efficiency of antigen presentation. The same method as that described in Example 6 was applied. The results are shown in Figure 7. When 2 µg of OVA that had been encapsulated into OML was administered, the amount of cytokine generated was almost equivalent to that in the case of a single administration of OVA (1,000 µg). Thus, it is considered that antigen presentation to the MHC class II in the case of using OML was approximately 500 times higher than that in the case of a single administration of OVA. Likewise, it is considered that antigen presentation to the MHC class I in the case of using OML was approximately 50 times higher than that in the case of a single administration of OVA. That is to say, antigen presentation can be extremely efficiently carried out by encapsulating an antigen into OML.

### Example 8:

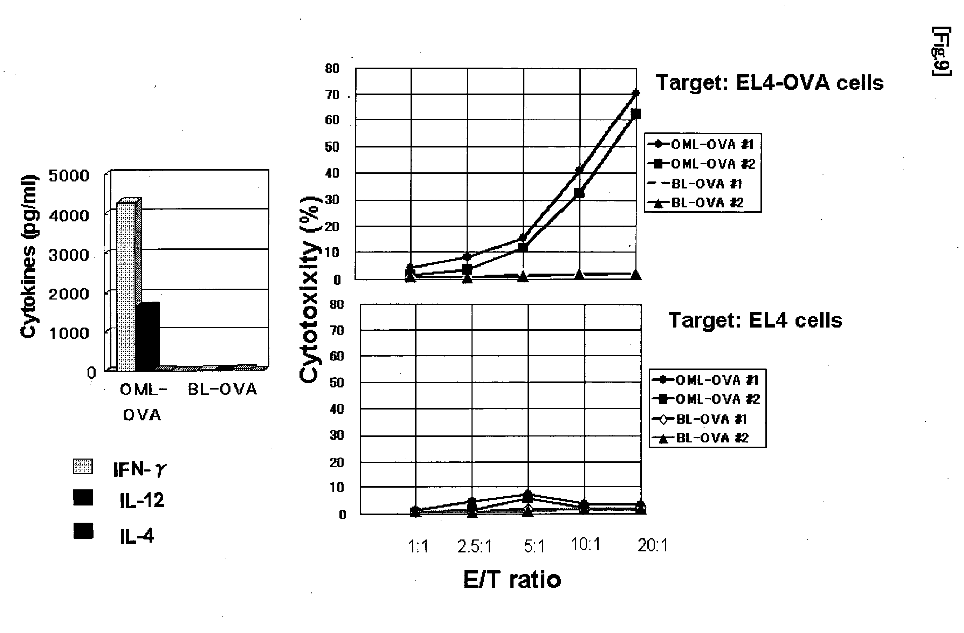

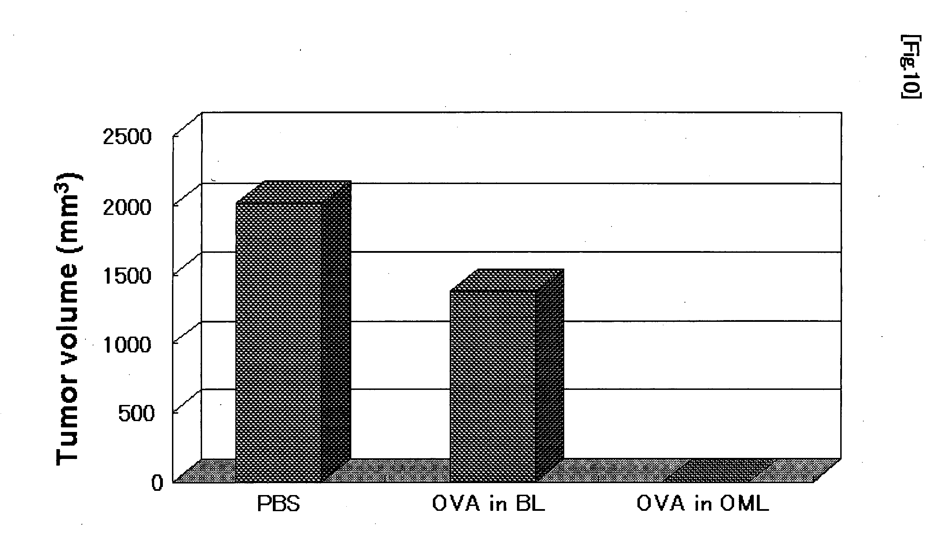

A C57BL/6 mouse was immunized with OVA/OML (approximately 1 µg of OVA) (subcutaneous administration twice at an interval of 1 week). Immunization schedule for activation of gut immunity is shown in Figure 8. 1 week after the final immunization, splenic cells were excised from the mouse and were then stimulated with an antigen. Thereafter, cytokine contained in the medium was measured (Figure 9). At the same time, cytotoxic ability was measured using EG7 cells (which were cells that introduced OVA genes into EL4 cells and presented OVA peptides on their own MHC class I molecules) as target cells (Figure 9). Significant generation of Th1 cytokine was observed in the mouse immunized with OVA-encapsulated OML, and significant cytotoxic ability was also observed. On the other hand, when the parent line EL4 was used as a target, no cytotoxic ability was observed in any cases. Accordingly, it can be said that antigen-specific CTL was induced.

### Example 9:

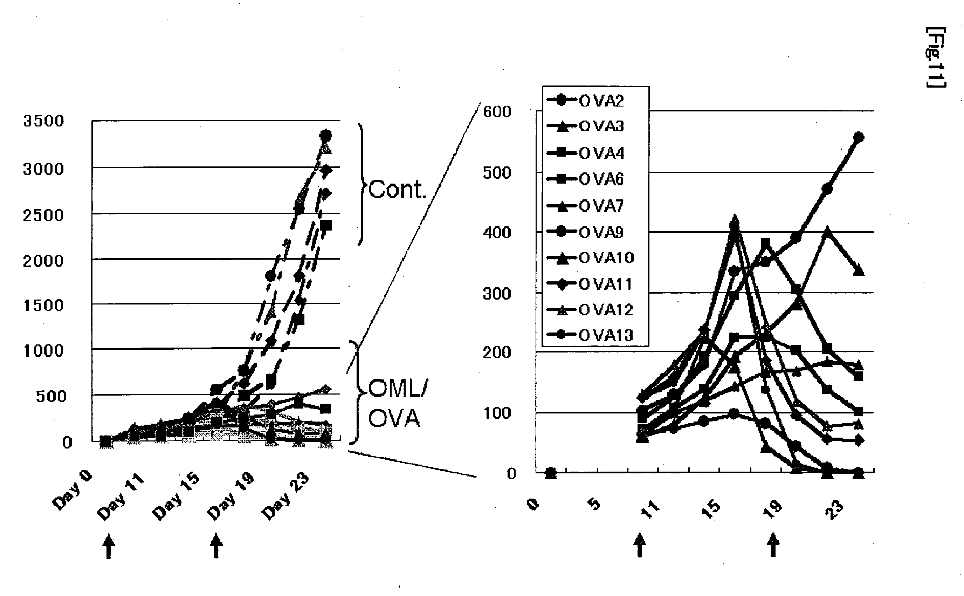

One week after the final immunization as described in Example 8, 1 x 10⁶ EG-7 was transplanted into the back of the immunized mouse. Three weeks later, a tumor volume was measured. The results are shown in Figure 10. In the case of a mouse immunized with OVA/OML, the survival of tumor was completely inhibited.

### Example 10:

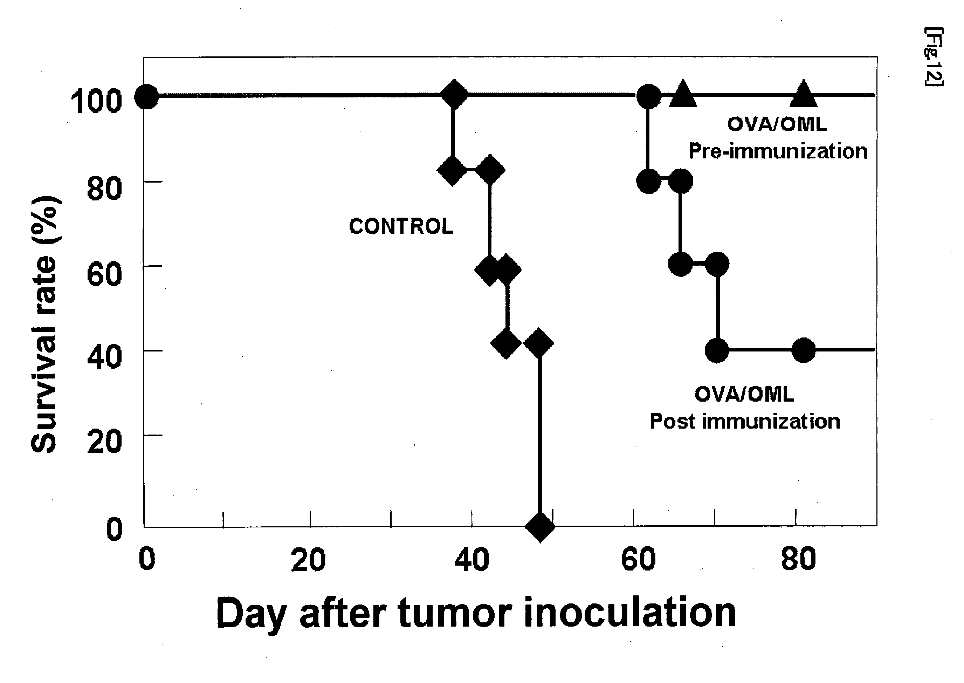

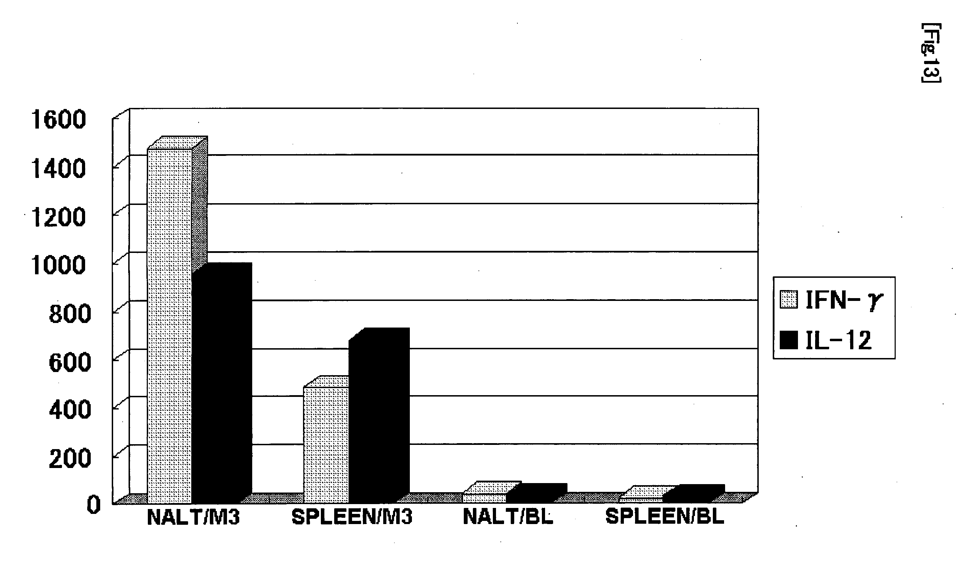

1 x 10⁶ EG-7 was transplanted into the back of each immunized mouse. On the 9^{th} day after the transplantation (tumor volume: approximately 100 mm³), the mouse was inoculated with OML/OVA (1 µg of OVA). Thereafter, a tumor volume was measured. The results are shown in Figure 11. In the OML/OVA administration group, involution of the tumor was observed in almost all mice, and in at least 3 cases, such tumor was completely involuted. The survival rate of the tumor-inoculated mice is shown in Figure 12. In the OML/OVA administration group, such survival rate was remarkably extended.

### Example 11:

OML/OVA (5 µg of OVA) was administered to the nasal cavity of each mouse total three times at intervals of 1 week. One week after the final administration, nasal-associated lymphatic tissues (NALT) and a spleen was excised from the mouse, and the amount of cytokine generated was measured after stimulation with an antigen. The results are shown in Figure 13. In the OML/OVA administration group, significant generation of Th1 cytokine was observed in NALT.

### Industrial Applicability

According to the present invention, it became possible to provide a liposome composition, which causes the MHC class I and class II molecules of an antigen-presenting cell to efficiently present an antigen peptide such as a cancer antigen. According to the present invention, a simple immunization method, which does not need an operation to collect cells from a patient body and then return them thereto, can be constructed. In particular, the liposome composition of the present invention enables encapsulation of a protein acting as a cancer antigen and direct administration of the protein to a patient. Thus, the liposome composition of the present invention is useful for vaccine therapy.

### Brief Description of the Drawings

Figure 1 shows the amount of IFN-γ generated from splenic cells derived from an MHC class I-restricted OVA peptide-specific TCR transgenic mouse.
Figure 2 shows the response of splenic cells derived from an MHC class II-restricted OVA peptide-specific TCR transgenic mouse (DO11.10) to a macrophage existing in an abdominal cavity, into which OVA has been incorporated.
Figure 3 shows induction of cytotoxic T cells by immunization with a Man3-liposome.
Figure 4 shows the amount of cytokine generated from intraperitoneal cells after an oligomannose-coated liposome (OML/OVA) or the like has been administered into an abdominal cavity.
Figure 5 shows the amount of cytokine generated from intraperitoneal cells after OML/OVA or LPS has been administered into an abdominal cavity.
Figure 6 shows the amount of cytokine generated in a case where CD8-positive T cells and CD4-positive T cells were collected from TCR transgenic mice OT-1 that recognize OVA257-264 (MHC class I) and TCR transgenic mice OT-2 that recognize OVA323-339 (MHC class II), respectively, and where the CD8-positive T cells, the CD4-positive T cells, and macrophages were subjected to a mixed culture.
Figure 7 shows the results obtained by comparing the case of single administration of OVA and the case of encapsulating OVA into OML and then administrating the OML, in terms of the efficiency of antigen presentation.
Figure 8 shows an immunization schedule for activation of gut immunity.
Figure 9 shows the results obtained by collecting splenic cells 1 week after the final immunization for activation of gut immunity, stimulating the cells with an antigen, and measuring the amount of cytokine contained in a medium, and the results obtained by measuring cytotoxic ability using EG7 cells as target cells.
Figure 10 shows the results obtained by transplanting EG-7 into the back of each mouse subjected to gut immunity 1 week after the final immunization and then measuring a tumor volume 3 weeks later.
Figure 11 shows the results obtained by measuring a tumor volume after inoculation of OML/OVA on the 9^{th} day after transplantation of EG-7 in the back of each mouse.
Figure 12 shows the survival rate of tumor-inoculated mice.
Figure 13 shows the results obtained by administering OML/OVA into the nasal cavity of each mouse, collecting nasal cavity-associated lymphatic tissues (NALT) and a spleen from the mouse 1 week after the final administration, and measuring the amount of cytokine generated after antigen stimulation.

## Claims

1. A liposome composition, which comprises an oligosaccharide-coated liposome and an antigen substance and is used to cause the MHC class I and class II molecules of an antigen-presenting cell to present an antigen peptide.

2. The liposome composition of claim 1 wherein the oligosaccharide is oligomannose.

3. The liposome composition of claim 1 or 2 wherein the oligosaccharide is mannopentaose or mannotriose.

4. The liposome composition of any of claims 1 to 3 wherein the antigen substance is a cancer antigen.

5. The liposome composition of any of claims 1 to 4 wherein the liposome is administered into an abdominal cavity, subcutis, or intranasal mucosa and is incorporated into an antigen-presenting cell such as a macrophage, and as a result, an antigen peptide is presented on the MHC class I and class II molecules.

6. The liposome composition of any of claims 1 to 5 which is used to induce cytotoxic T lymphocytes (CTL).
